(19) Europäisches Patentamt
European Patent Office
Office européen des brevets

(11) EP 2 060 271 A1

# (12) EUROPEAN PATENT APPLICATION

published in accordance with Art. 153(4) EPC

(43) Date of publication:
**20.05.2009 Bulletin 2009/21**

(21) Application number: **08720571.2**

(22) Date of filing: **24.03.2008**

(51) Int Cl.:
***A61K 47/36*** (2006.01) ***A61K 9/127*** (2006.01)
***A61K 39/00*** (2006.01) ***A61K 39/35*** (2006.01)
***A61K 47/18*** (2006.01) ***A61K 47/24*** (2006.01)
***A61K 47/28*** (2006.01) ***A61P 35/00*** (2006.01)
***A61P 37/04*** (2006.01) ***A61P 43/00*** (2006.01)

(86) International application number:
**PCT/JP2008/000692**

(87) International publication number:
**WO 2008/114516 (25.09.2008 Gazette 2008/39)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **22.03.2007 JP 2007075615**

(71) Applicants:
- **Kibun Food Chemifa Co., Ltd.**
  **Tokyo 1048553 (JP)**
- **Kitasato Gakuen**
  **Minato-ku**
  **Tokyo 1088641 (JP)**

(72) Inventors:
- **KUMAZAWA, Yoshio**
  **Sagamihara-shi**
  **Kanagawa 228-8555 (JP)**
- **TAKIMOTO, Hiroaki**
  **Sagamihara-shi**
  **Kanagawa 228-8555 (JP)**
- **INOUE, Joe**
  **Sagamihara-shi**
  **Kanagawa 228-8555 (JP)**
- **KUBOTA, Masahiro**
  **Tokyo 104-8553 (JP)**
- **NOZAWA, Takashi**
  **Tokyo 104-8553 (JP)**
- **MURATA, Katsumi**
  **Tokyo 104-8553 (JP)**

(74) Representative: **Zimmermann & Partner**
**Postfach 330 920**
**80069 München (DE)**

## (54) LIPOSOME AND IMMUNOSTIMULATING COMPOSITION COMPRISING THE SAME

(57) It is an object to enhance immunopotentiating activity of a glycosphingolipid.

A liposome, comprising at least one type of glycosphingolipid represented by the following Formula (1) inside and/or outside a lipid membrane:

Formula (1)

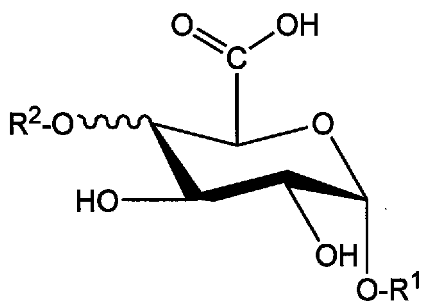

EP 2 060 271 A1

**Description**

Technical Field

The present invention relates to a liposome containing a glycosphingolipid with a specific structure inside and/or outside the lipid membrane and an immunopotentiating composition containing the liposome.

Background Art

The present inventors have revealed that glycosphingolipids separated from specific bacteria of the genus *Sphingomonas* have immunopotentiating activity and the like (Patent documents 1 to 5). However, it cannot be said that such immunopotentiating activity and the like are always sufficient, and thus further activation has been required.
Patent document 1 International Publication WO92/1298
Patent document 2 JP Patent Publication (Kokai) No. 11-43437 A (1999)
Patent document 3 JP Patent Publication (Kokai) No. 2000-51676 A
Patent document 4 JP Patent Publication (Kokai) No. 2002-010797 A
Patent document 5 JP Patent Publication (Kokai) No. 2005-263797 A

Disclosure of the Invention

Objects to be Achieved by the Invention

The objects of the present invention are to overcome the aforementioned problems and to further enhance the immunopotentiating activity of the above glycosphingolipids.

Means for Attaining the Objects

Under the above circumstances, the present inventors have discovered that the aforementioned objects can be attained by the following means.

(1) A liposome, comprising at least one type of glycosphingolipid represented by the following Formula (1) inside and/or outside a lipid membrane: Formula (1)

[Formula 1]

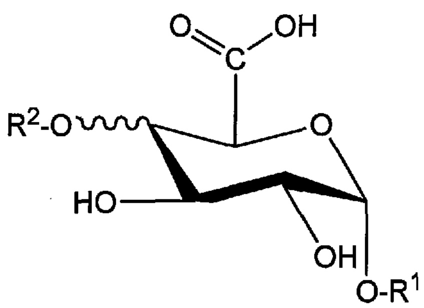

(wherein $R^1$ represents Formula (1-1):

Formula (1-1)

[Formula 2]

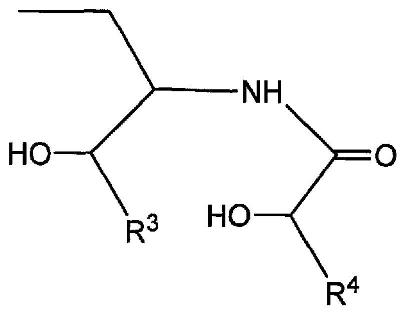

(wherein $R^3$ represents alkyl group or alkenyl group and $R^4$ represents alkyl group) and $R^2$ represents a hydrogen atom, α-galactose, α-glucose, α-mannose, α-glucosamine, β-glucosamine, or a combination of one or more thereof.)
(2) The liposome according to (1), wherein $R^2$ in Formula (1) represents a hydrogen atom, α-galactose, α-glucose, α-mannose, α-glucosamine, β-glucosamine, or a combination of two or three thereof.
(3) The liposome according to (1), wherein $R^2$ in Formula (1) represents a hydrogen atom or the following $R^{62}$, $R^{63}$, $R^{64}$, or $R^{65}$.

[Formula 3]

$R^{62}$ :

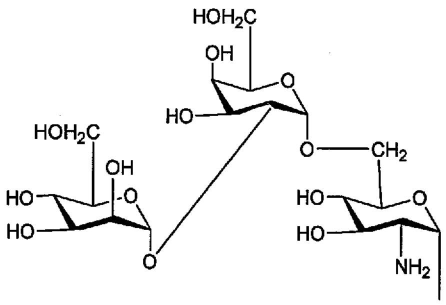

$R^{64}$ :

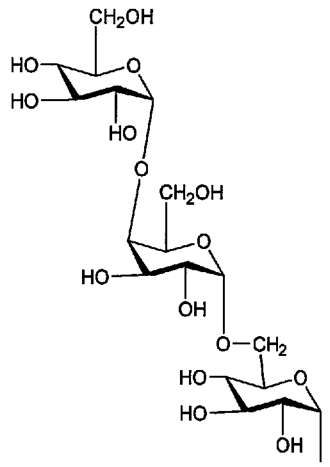

$R^{63}$ :

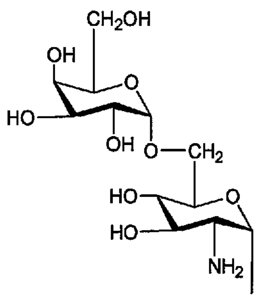

$R^{65}$:

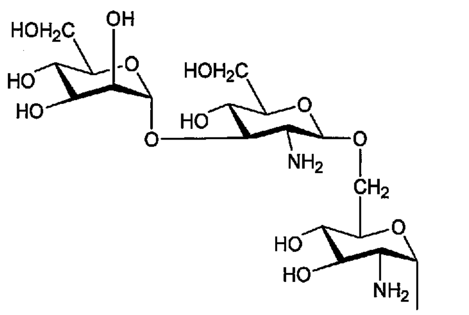

(4) The liposome according to (1), wherein $R^2$ in Formula (1) represents a hydrogen atom.
(5) The liposome according to any one of (1) to (4), wherein $R^1$ in Formula (1) represents the following $R^{51}$, $R^{52}$, $R^{53}$, $R^{54}$, $R^{55}$, $R^{56}$, $R^{57}$, $R^{58}$, $R^{59}$, $R^{70}$, $R^{71}$, $R^{72}$, $R^{73}$, $R^{74}$, $R^{75}$, $R^{76}$, $R^{77}$, or $R^{78}$.

[Formula 4]

R[51] :

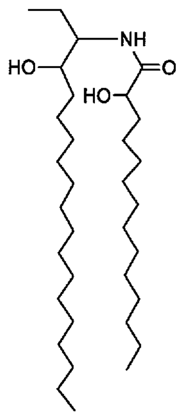

R[52] :

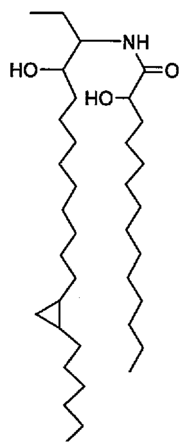

R[53] :

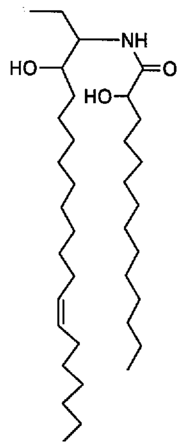

[Formula 5]

R[54] :

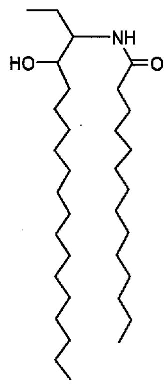

R[55] :

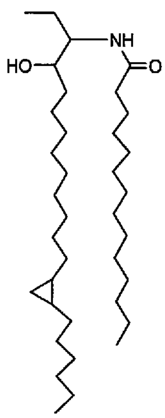

R[56] :

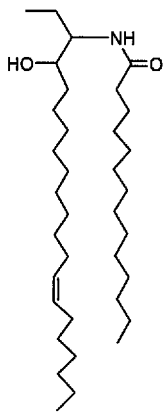

R[57] :

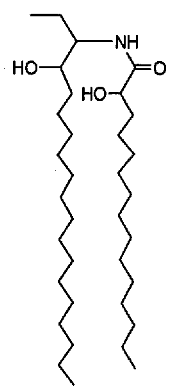

R[58]:

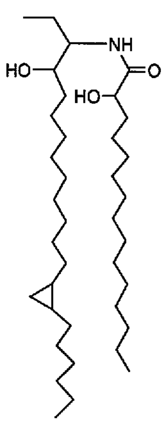

R[59] :

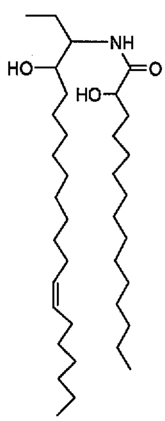

[Formula 6]

$R^{70}$ :

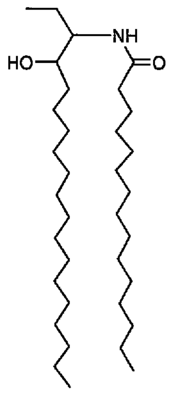

$R^{71}$ :

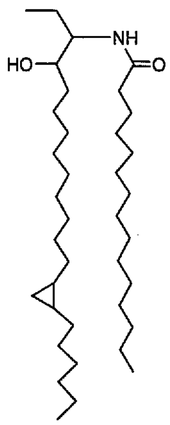

$R^{72}$ :

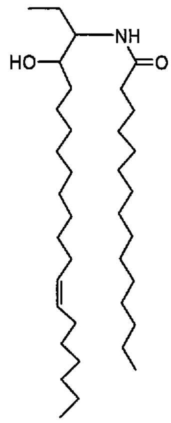

$R^{73}$ :

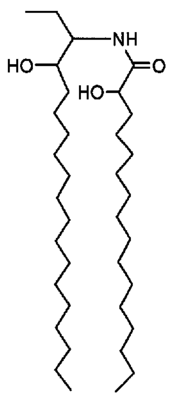

$R^{74}$ :

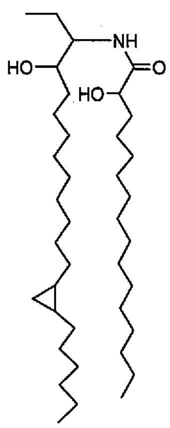

$R^{75}$ :

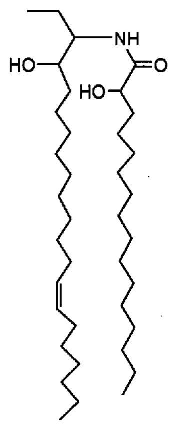

[Formula 7]

$R^{76}$:

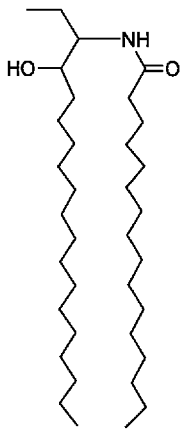

$R^{77}$:

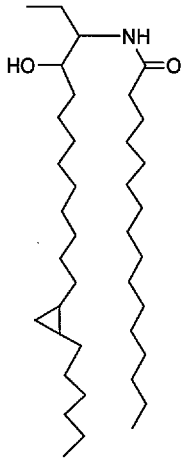

$R^{78}$:

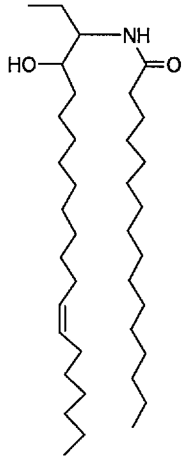

(6) The liposome according to any one of (1) to (5), wherein the lipid membranes is composed of a cationic lipid.
(7) The liposome according to any one of (1) to (5), wherein the lipid membrane is composed of a cationic lipid in the molar ratio of 0.4 or more.
(8) The liposome according to (6) or (7), wherein at least one type of the cationic lipid is 1,2-dioleoyl-3-(trimethyl ammonium)propane.
(9) The liposome according to any one of (1) to (7), wherein the lipid membrane is composed of 1,2-dioleoyl-3-(trimethyl ammonium)propane, 3β-[N-(dimethylaminoethane)carbamoyl]cholesterol, and 1,2-dioleoyl-sn-glycero-3-phosphatidylethanolamine in the molar ratio of 0.75 or more.
(10) The liposome according to any one of (1) to (9), further comprising an antigen inside and/or outside the lipid membrane.
(11) An immunopotentiating composition, comprising the liposome according to any one of (1) to (10).
(12) A composition for accelerating IFN-γ production, comprising the liposome according to any one of (1) to (10).
(13) A composition for accelerating IL-4 production, comprising the liposome according to any one of (1) to (10).
(14) A composition for accelerating IL-12 production, comprising the liposome according to any one of (1) to (10).
(15) A composition for accelerating antibody production, comprising the liposome according to any one of (1) to (10).
(16) An antitumor composition, comprising the liposome according to any one of (1) to (10).
(17) A composition for NKT cell activation, comprising the liposome according to any one of (1) to (10).
(18) A composition for dendritic cell activation, comprising the liposome according to any one of (1) to (10).
(19) An antiviral composition, comprising the liposome according to any one of (1) to (10).
(20) An antiallergic composition, comprising the liposome according to any one of (1) to (10).

Effects of the Invention

The present invention makes it possible to further enhance the immunopotentiating activity of a glycosphingolipid (s) represented by Formula (1) (hereinafter, referred to as "GSL(s)") by preparing a liposome that contains at least one type of GSL inside and/or outside the lipid membrane.

Brief Description of the Drawings

[Fig. 1] Fig. 1 shows the amount of a liposome introduced by $CD11c^+$ dendritic cells prepared from splenocytes.
[Fig. 2] Fig. 2 shows tumor shrink due to addition of a liposome.
[Fig. 3] Fig. 3 shows the results of observing the liposomes of the present invention using an electron microscope.
[Fig. 4] Fig. 4 shows an enlarged view of one of the liposomes in Fig. 3.
[Fig. 5] Fig. 5 shows the results for TLC of lipid extracted from Liposome B.

Preferred Embodiments of the Invention

Hereafter, the present invention is described in detail. In the followings, the content of the invention is to be described specifically. In the specification of the present application "--- to ---" is used in the meaning of including numerical values described before and after thereof as a lower limit value and an upper limit value.

The term "liposome(s)" in the present invention refers to a lipid aggregating in such a manner that the hydrophobic portions of the lipid are oriented inward to each other (and the same may also be referred to as "lipid membrane(s)" in this description) and are composed of inner aqueous layers.

Furthermore, the expression, "containing at least one type of glycosphingolipid represented by Formula (1) inside and/or outside a lipid membrane," used in this description means that GSLs are present in any one of on the surface, within the lipid membrane and within the liposome. In general, a part of or the whole of GSL is contained in the lipid membrane of the liposome.

The lipid membranes according to the present invention may have either a monolayer structure or a multilayer structure.

According to the present invention, as described above, it has been surprisingly discovered that the immunopotentiating activity of GSLs can be enhanced by preparing liposome formation containing GSLs. Glycolipids is used as lipid components of lipid membranes upon liposome formation; however, it is rare for glycolipids themselves to be used as target substances to be caused to undergo liposome formation as is the case of the present invention. Furthermore, it is extremely noteworthing that liposome formation by GSLs significantly enhances the immunopotentiating activity of the GSLs.

First, a GSL to be employed in the present invention is described in detail. A GSL to be employed in the present invention is a glycosphingolipid represented by the following Formula (1).

Formula (1)

[Formula 8]

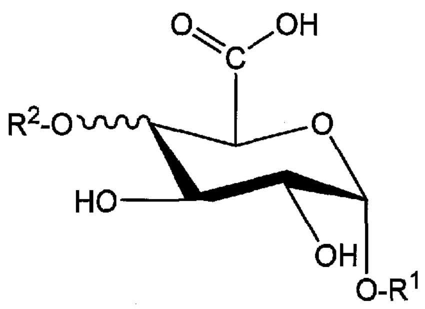

(wherein $R^1$ represents Formula (1-1)

Formula (1-1)

[Formula 9]

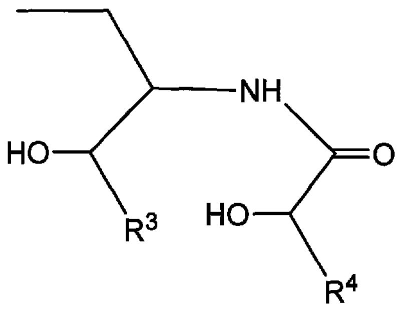

(wherein $R^3$ represents alkyl group or alkenyl group and $R^4$ represents alkyl group) and $R^2$ represents a hydrogen atom, α-galactose, α-glucose, α-mannose, α-glucosamine, β-glucosamine, or a combination of one or more thereof.)

Alkyl group represented by $R^3$ included in $R^1$ of Formula (1) may contain cycloalkyl group and the cycloalkyl group may be present at the alkyl terminus or in the chain of $R^3$. A preferable example of cycloalkyl group is cyclopropane group. Alkyl group represented by $R^3$ has preferably 13 to 23 of carbon atoms, and more preferably 15, 16, 17, 18, 19, 20, or 21 of carbon atoms. Moreover, alkyl group or alkenyl group represented by $R^3$ is preferably of the substituted or unsubstituted straight chain type. A double bond in alkenyl group may be present at any position.

In contrast, alkyl group represented by $R^4$ has preferably 10 to 20 of carbon atoms, more preferably 10, 11, 12, 13, 14, 15, or 16 of carbon atoms, and further preferably 10, 11, 12, 13, 14, or 15 carbon atoms of carbon atoms. Furthermore, alkyl group represented by $R^4$ is preferably of the substituted or unsubstituted straight chain type.

More preferably, $R^1$ represents any of the following $R^{51}$ - $R^{78}$.

[Formula 10]

$R^{51}$ :

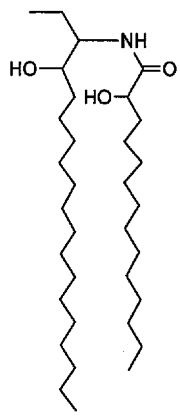

$R^{52}$ :

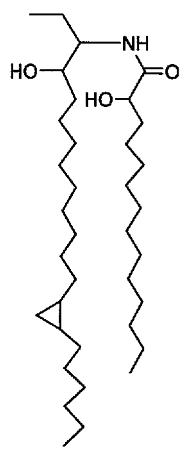

$R^{53}$ :

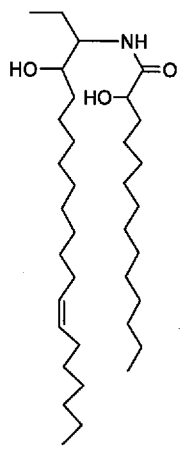

[Formula 11]

$R^{54}$ :

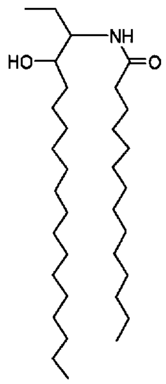

$R^{55}$ :

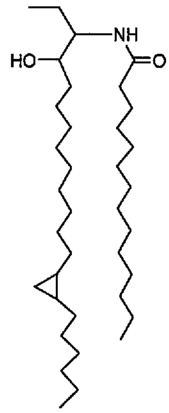

$R^{56}$ :

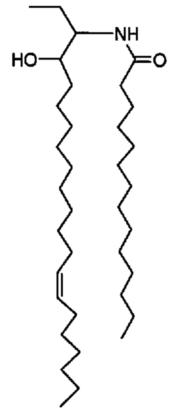

$R^{57}$ :

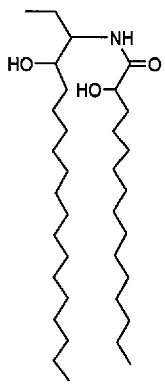

$R^{58}$ :

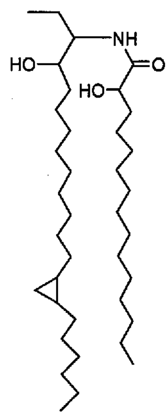

$R^{59}$ :

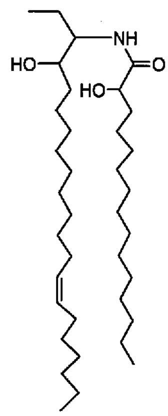

[Formula 12]

$R^{70}$ :

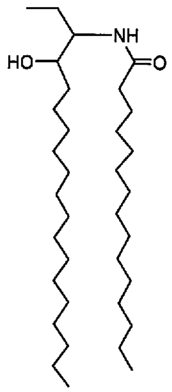

$R^{71}$ :

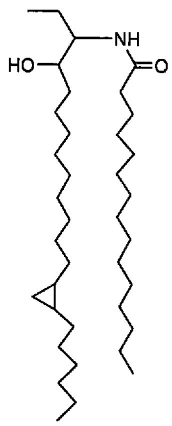

$R^{72}$ :

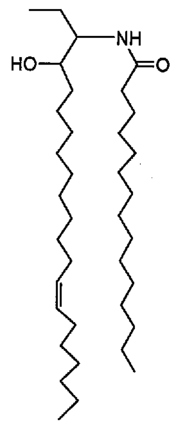

$R^{73}$ :

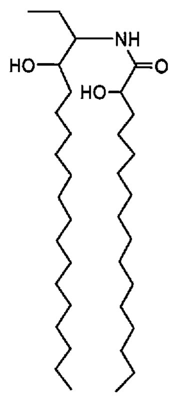

$R^{74}$ :

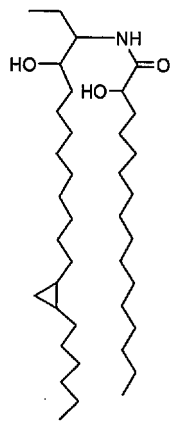

$R^{75}$ :

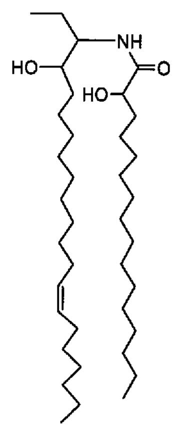

[Formula 13]

$R^{76}$ :

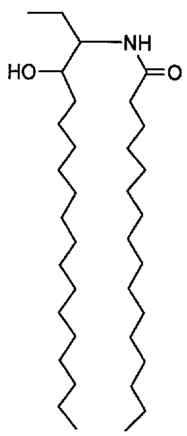

$R^{77}$ :

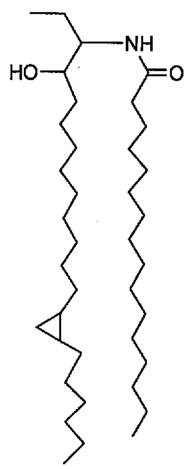

$R^{78}$ :

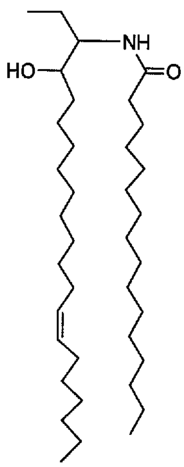

Furthermore, $R^1$ in Formula (1) preferably represents the conformation represented by the following Formula

(1-2):

Formula (1-2)

[Formula 14]

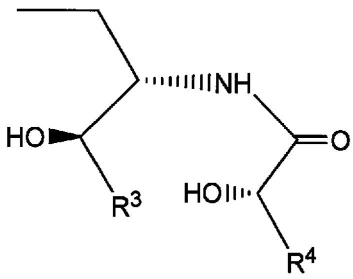

wherein $R^3$ and $R^4$ in Formula (1-2) are the same as defined above in Formula (1-1). Thus, the aforementioned $R^{51}$ - $R^{78}$ having such conformations are more preferable.

Meanwhile in a structure represented by Formula (1), $R^2$ represents preferably a hydrogen atom or a group that is α-galactose, α-glucose, α-mannose, α-glucosamine, or β-glucosamine, or comprised of two or three combinations thereof, more preferably a hydrogen atom, the following $R^{62}$, $R^{63}$, $R^{64}$, or $R^{65}$, and further more preferably a hydrogen atom. Because of employment of a structure wherein $R^2$ is a hydrogen atom, the effects of various immunopotentiating activities tend to be particularly improved.

[Formula 15]

$R^{62}$ :

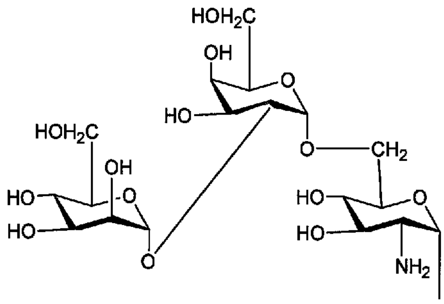

$R^{64}$ :

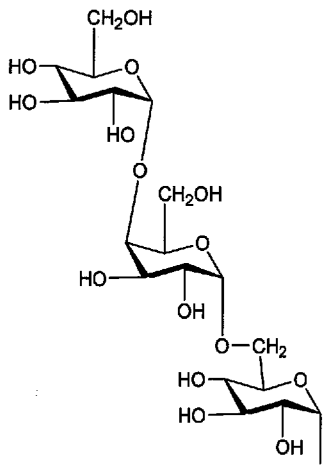

$R^{63}$ :

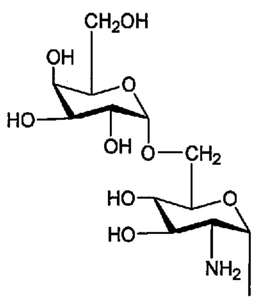

$R^{65}$ :

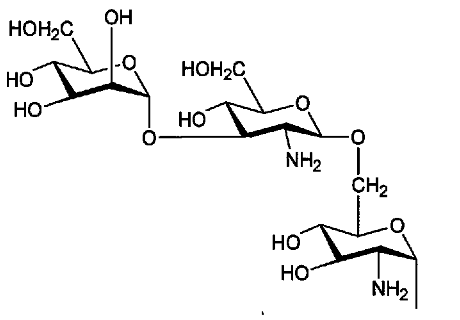

Furthermore, in the present invention, a single type or two or more types of glycosphingolipid may be used. When two or more types of glycosphingolipid are contained in combination, the proportion of each component is not particularly limited.

GSLs can be obtained by extracting glycosphingolipids from bacteria having the same. For example, methods disclosed in WO92/12986, JP Patent Publication (Kokai) No. 2002-10797 A, or JP Patent Publication (Kokai) No. 2005-263797 A can be employed. Since glycosphingolipids are contained in bacteria of the family *Sphingomonas,* the glycosphingolipid represented by Formula (1) can be extracted and thus obtained from any of the bacteria of the family *Sphingomonas.* Here, the bacteria belonging to the family *Sphingomonas* include bacteria that have been generally said to belong to the genus *Sphingomonas* and bacteria that are classified as belonging to substantially the same family of *Sphingomonas* bacteria. For example, any bacterial strains described in Microbiol. Immunol., 2000, 44, 563-575 can be used in the present invention.

GSLs are insoluble in acetone. Accordingly, bacteria are preferably washed with acetone before extraction. An alcoholic solvent such as methanol or a mixed solvent comprising an alcoholic solvent and a polar solvent such as chloroform is preferably used for extraction of GSLs from the viewpoint of yield. Other types of solvents may be used as long as such solvents are capable of dissolving glycosphingolipids.

In the present invention, particularly the following GSLs listed in the following table can be preferably used. In the table, a sphingolipid portion of a GSL is one or more types of the above $R^{51}$ - $R^{78}$. Moreover, it goes without saying that GSLs to be used in the present invention are not limited to those obtained from the bacterial strains listed as follows.

[Table 1]

| Sample name | Source | $R^2$ in Formula (1) |
|---|---|---|
| GSL-1 | *S. paucimobilis* | H(α) |
| GSL-2 | *S. paucimobilis* | $R^{62}$ |
| GSL-3 | *S. capsulata* | $R^{63}$ |
| GSL-4 | *S. adhaesiva* | $R^{64}$ |
| GSL-5 | *S. sp. MK346* | $R^{65}$ |

(continued)

| Sample name | Source | $R^2$ in Formula (1) |
|---|---|---|
| GSL-6 | *S. yanoikuyae* | H(α) |
| GSL-7 | *S. yanoikuyae* | H(β) |
| GSL-8 | *S. wittichii* | H(α) |
| GSL-9 | *S. wittichii* | H(β) |
| GSL-10 | *S. macrogoltabidus* | H(α) |
| GSL-11 | *S. macrogoltabidus* | H(β) |
| GSL-12 | *S. terrae* | H(α) |
| GSL-13 | *S. terrae* | H(β) |

Furthermore, when glycosphingolipid mixtures are obtained, components thereof can be separated from one another in accordance with a technique known in the art, such as the method disclosed in JP Patent Publication (Kokai) No. 2005-263797 A. Specifically, glycosphingolipids can be completely separated by chromatography. Glycosphingolipids are each eluted in the order of (1) GSLs: 1 and 6 - 13, (2) GSL: 3, and (3) GSLs: 2, 4, and 5 when a mixed solution of chloroform and methanol is used as an eluate. Furthermore, GSLs: 2, 4, and 5 are produced by different bacterial strains, theose GSLs can be separated extremely conveniently. Conditions for separation via chromatography, such as a filler, an eluate, a rate of elution, pressure, and temperature, can be adequately regulated. Alternatively, a reagent that selectively reacts with only a specific substance contained in the glycosphingolipid mixture may be caused to act on such substance, so as to prepare a derivative of such substance, and separation can be carried out with the utilization of chemical or physical properties of such derivative.

Furthermore, GSLs can be synthesized by combining conventional synthesis methods. For example, a sugar portion and a sphingosine portion are synthesized in advance or extracted from the bacteria to form amide bonds. Thus, GSLs can be prepared.

Next, the liposome of the present invention and techniques for liposome formation are as described below.
The diameter ("mean volume particle diameter") of the liposome of the present invention generally ranges from 20 nm to 2000 nm and preferably ranges from 20 nm to 800 nm. With the use of such range, the effects can be easily exerted when the liposome is used as a pharmaceutical compound (medicinal preparation).
The "mean volume particle diameter" of the liposome can be found based on the principle of dymamic light scattering or the like (see D. D. Lasic, "liposomes: from basic to applications," Elsevier Science Publishers, pp. 1-171 (1993)).

In the liposome of the present invention, preferably the proportion of particles having a mean volume particle diameter or the mean volume particle diameter ± 5% accounts for 50% or more of the whole particles.

The GSL content in the liposome of the present invention is not particularly limited and preferably ranges from 0.01% to 30% with respect to the total lipid content (excluding GSL content) of the lipid membrane.

Examples of lipids composing the lipid membrane of the liposome of the present invention are not particularly limited, as long as they can form liposomes. Synthetic and natural lipids can be broadly used. For example, the following lipids can be used.

Examples of such lipids include N-[1-(2,3-dioleyloxy)propyl]-N,N,N-trimethylammonium chloride (DOTMA), N-(α-trimethylammonioacetyl)-didodecyl-D-glutamate chloride (TMAG), 2,3-Dioleyloxy-N-[2(Spermine carboxamido) ethyl]N,N-Dimethyl-1-propanammonium trifluoroacetate (DOSPA), 1,2-dimyristyloxypropyl-3-dimethyl-hydroxy ethyl ammonium bromide (DMRIE), dioctadecylamidoglycyl spermine (DOGS), dimethyl dioctadecyl ammonium bromide (DDAB), N, $N^1$, $N^2$, $N^3$-Tetramethyl-tetrapalmitylspermine (TM-TPS), 1,2-dioleoyl-3-(trimethylammonium) propane (DOTAP), phosphatidylcholine (e.g., soybean phosphatidylcholine, egg yolk phosphatidylcholine, dilauroyl phosphatidylcholine, dimyristoylphosphatidylcholine, dipalmitoylphosphatidylcholine (DPPC), or distearoylphosphatidylcholine), phosphatidylethanolamine (e.g., 1,2-dioleoyl-sn-glycero-3-phosphatidylethanolamine (DOPE), dilauroylphosphatidylethanolamine, dimyristoylphosphatidylethanolamine, dipalmitoylphosphatidylethanolamine (DPPE), or distearoylphosphatidylethanolamine), phosphatidylserine (e.g., dilauroylphosphatidylserine, dimyristoylphosphatidylserine, dipalmitoylphosphatidylserine, or distearoylphosphatidylserine), phosphatidic acid, phosphatidylglycerol (e.g., dilauroylphosphatidylglycerol, dimyristoylphosphatidylglycerol, dipalmitoylphosphatidylglycerol (DPPG), or distearoylphosphatidylglycerol), phosphatidylinositol (e.g., dilauroylphosphatidylinositol, dimyristoylphosphatidylinositol, dipalmitoylphosphatidylinositol, or distearoylphosphatidylinositol), lysophosphatidylcholine, sphingomyelin, egg-yolk lecithin, soybean lecithin, digalactosyldiglycerides (e.g., digalactosyl dilauroyl glyceride, digalactosyl dimyristoyl glyceride, digalactosyl dipalmitoyl glyceride, or digalactosyl distearoyl glyceride), or galactosyldiglycerides (e.g., galactosyl dilauroyl glyceride,

galactosyl dimyristoyl glyceride, galactosyl dipalmitoyl glyceride, or galactosyl distearoyl glyceride), galactosyl cerebroside, lactosyl cerebroside or ganglioside, cholesterol, cholesterol hemisuccinate, 3β-[N-(dimethylaminoethane)carbamoyl]cholesterol (DC cholesterol), ergosterol or lanosterol, dialkyl dimethylammnonium amphiphiles, polyglycerol alkyl ether, polyoxyethylene alkyl ether, alkyl glycoside, alkyl methyl glucamide, alkyl sucrose ester, dialkyl polyoxyethylene ether, didalkyl polyglycerol ether, polyoxyethylene-polylactic acid, long-chain alkyl amines (e.g., tetradecylamine, hexadecylamine, or stearyl amine), or long-chain fatty acid hydrazides (e.g., myristic acid hydrazide, palmitic acid hydrazide or stearic acid hydrazide).
These lipids can be used independently or in combination of two or more types thereof.

In particular, a lipid membrane in the present invention preferably contains cationic lipids for improvement of immunopotentiating activity. The molar ratio of such cationic lipids to all of the lipids composing the lipid membrane is more preferably 0.4 or more.
Examples of cationic lipids include DOTAP, DC cholesterol, DOTMA, stearyl amine, TMAG, DOSPA, DMRIE, DOGS, DDAB, and TM-TPS. The cationic lipid preferably contains at least DOTAP.
Particularly in the present invention, the molar ratio of DOTAP, DC cholesterol, and DOPE to all of the composing the lipid membrane is more preferably 0.75 or more and the molar ratio of DOTAP, DC cholesterol, and DOPE to the same is further more preferably 0.90 or more.
It is preferable that such lipid compositions tend to result in higher immunopotentiating activity.

Furthermore, in the present invention, other substances can also be added as lipid membrane components if necessary in addition to the above lipids. Specific examples of such substances include stabilizers, various functional substances, and agents for charge adjustment for lipid membranes. These "other" substances are preferably contained within a range between approximately 0.0001% and 30% with respect to the total lipid content composing the liposome.

Various substances may be encapsulated in the liposome of the present invention within the scope of the present invention. Examples of substances to be encapsulated are not particularly limited and include substances to be used for pharmaceutical products. Specific examples of such substances include contrast compounds, antitumor compounds, antioxidative compounds, antimicrobial compounds, antiinflammatory compounds, compounds for blood circulation promotion, whitening compounds, compounds for skin problems, antiaging compounds, hair-growth-stimulating compounds, moisturizing compounds, hormonal agents, vitamins, dyes, and proteins. A substance to be encapsulated is desirably a water-soluble compound or is in a water-soluble salt form or is a water-soluble form prepared by addition of a solubilizing agent. Such substance may be a hydrophobic or a lipid-soluble compound.
Particularly in the present invention, encapsulation of an antigen is preferred. This is preferable because of the presence of an advantage such that the effect of enhancing an antigen-specific immunoresponse can be induced by encapsulation of an immunogen. Examples of such antigen to be encapsulated are not particularly limited and include antigens, to be used for vaccination, tumor antigens, autoantigens of autoimmune diseases, and allergens.

The liposome of the present invention can be produced according to a known production method. For example, a method disclosed in Bangham, A.D., Standish, M.M. and Watkins, J.C. "Diffusion of univalent ions across the lamellae of swollen phospholipids." (1965) J. Mol. Biol. 13, 238-252 or the like can be employed. More specifically, the liposome can be produced according to the following procedures, however, the method for producing the liposome of the present invention is not limited by such description.

1) GSLs are dissolved in a solvent. At this time, preferably lipids can be dissolved in the solvent having low boiling points. Specific examples of organic solvents include chloroform and methanol. Furthermore, ultrasonication is preferably carried out after heating at 30°C to 70°C.
2) Components (excluding GSLs) of the lipid membrane are dissolved or suspended in an organic solvent. An organic solvent that is preferably used herein is characterized in that lipids can be dissolved therein and the solvent has a low boiling point. Specific examples of such organic solvent include chloroform, methanol, and ethanol.
3) The solution obtained in 1) above is mixed with the solution obtained in 2) above to remove the solvents, thereby the lipid membrane is formed.
4) An aqueous solvent (preferably, buffer) is added to the lipid membranes obtained in 3) above and then the mixture is agitated. Thus liposomes suspended in the aqueous solvent can be obtained.

The aqueous solvent (suspension) containing the liposomes obtained above is further filtered, so that the particle size of the liposomes can be equalized. Furthermore, the water-soluble solvent containing the liposomes is subj ected to ultracentrifugation, so that the liposomes can be separated as a precipitate. The thus separated liposomes may be washed if necessary.

Liposome formation can be confirmed by a known method. For example, liposome formation is confirmed by measuring the particle size of a substance (prepared by a known method for liposome formation) by light scattering and then confirming that the liposome according to the present invention is formed when the mean particle size is 2000 nm

or less. Furthermore, "particles" used herein are not necessarily required to be completely spherical.

The liposome of the present invention has an effect of further enhancing immunopotentiating activity. The liposome can enhance: particularly, acceleration of IFN-γ production, acceleration of IL-4 production, acceleration of IL-12 production, acceleration of antibody production, antitumor effects, NKT cell activity, dendritic cell activity, effects of enhancing resistance to infection, antiviral effects, acceleration of IL-10 production, NK cell activity, antiallergic effects, acceleration of IL-6 production, and acceleration of NO production; can further enhance acceleration of IFN-γ production, acceleration of IL-4 production, acceleration of IL-12 production, acceleration of antibody production, antitumor effects, NKT cell activity, dendritic cell activity, effects of enhancing resistance to infection, and antiviral effects; can further more enhance acceleration of IFN-γ production, acceleration of IL-4 production, acceleration of IL-12 production, acceleration of antibody production, antitumor effects, NKT cell activity, dendritic cell activity, and effects of enhancing resistance to infection; and can particularly enhance acceleration of IFN-γ production, acceleration of IL-4 production, acceleration of IL-12 production, acceleration of antibody production, and antitumor effects.

Examples of functions accelerated by acceleration of IFN-γ production include Th1 induction and macrophage activation that are accelerated by IFN-γ.

Examples of functions accelerated by acceleration of IL-4 production include Th2 induction and induction of antibody class switching.

The term "acceleration of antibody production" in this description refers to the effects of enhancing antigen-specific immunoresponse and adjuvant effects.

The term "antitumor effects" in this description is meant to include, for example, helper T cell activation and killer T cell activation.

The term "NKT cell(s)" in this description is meant to include human $V\alpha 24^+$NKT cell(s) and mouse $V\alpha 14^+$NKT cell(s). Furthermore, the term "NKT cell activation" is meant to include enhancement of cellular cytotoxicity, enhancement of cytokine production, and acceleration of NKT cell proliferation.

The term "dendritic cell activation" in this description is meant to include, for example, enhancement of antigen-presenting capacity.

The term "the effects of enhancing resistance to infection" in this description is meant to include, for example, helper T cell activation and killer T cell activation. The compositions for enhancing resistance to infection according to the present invention are preferably applied to salmonella infection and tuberculosis.

The term "antiviral effects" in this description is meant to include, for example, enhancement of I-type interferon production, helper T cell activation, and killer T cell activation. The antiviral compositions according to the present invention are preferably applied to Herpesviridae infections such as cytomegalovirus infection or herpesvirus infection.

The term "NK cell activation" in this description is meant to include, for example, damage to infected cells.

The term "antiallergic effects" in this description is meant to include, for example, suppression of eosinophilic infiltration, mast cell suppression activity, suppression of IgE-production, and suppression of chemical transmitter release. Furthermore, the antiallergic compositions of the present invention tend to have mild side effects, and thus, application thereof to pollenosis, asthma bronchiale, or the like is particularly useful.

When the liposome and the liposome-containing composition of the present invention are used as pharmaceutical preparations, quasi-drugs, or active ingredients thereof, they can be preferably administered as pharmaceutical compositions that can be produced by a method known by the skilled person in the art. Examples of pharmaceutical compositions include tablets, capsules, powders, subtle granules, granules, liquids, and syrups. Such pharmaceutical compositions can be produced with the addition of pharmacologically or pharmaceutically acceptable additives. Examples of pharmacologically or pharmaceutically acceptable additives include excipients, disintegrators or disintegration assistants, binders, lubricants, coating agents, dyes, diluents, bases, solubilizers or solubilization assistants, isotonizing agents, pH regulators, stabilizers, propellants, and adhesives. The pharmaceutical compositions may also comprise 1 or more ingredients having other effects within the scope of the present invention. The dose of the pharmaceutical preparations according to the present invention is not particularly limited, and it can be adequately determined in accordance with the type of active ingredient or other conditions. Such dose can be adequately increased or decreased in accordance with a variety of general factors such as the body weight or age of the patient, the type or symptom of the disease, or the route of administration. In general, the pharmaceutical compositions can be administered in amounts ranging from 0.001 mg to 1000 mg, and preferably 0.01 mg to 100 mg, per adult per day. The route of administration is not particularly limited, and administration can be made intravenously via injection or infusion or orally.

Examples

The present invention is hereafter described in greater detail with reference to the examples. The materials, the amounts used, the proportions, the procedures, the processes, and other conditions described in the following examples can be adequately altered within the scope of the present invention. Accordingly, the scope of the present invention is not limited to the examples.

GSL-7 shown in Table 1 was used in the examples. The GSLs were separated in accordance with the methods disclosed in WO 92/12986, JP Patent Publication (Kokai) No. 2002-010797, and JP Patent Publication (Kokai) No. 2005-263797 A.
Furthermore, in each table in the examples: "control" represents the result of an comparative example in which the experiment was conducted under the same conditions except that neither liposome nor GSL-7 (P20) not undergoing liposome formation was added; and "GSL-7(P20)" represents the result of an comparative example in which the experiment was conducted under the same conditions except that only GSL-7(P20) not undergoing liposome formation was added.

Example 1 Induction of IFN-γ production from splenocytes

(Preparation of liposomes)

GSL-7 was dissolved in physiological saline for injection (Otuka Pharmaceutical) supplemented with Polysorbate-20 (Wako) (0.5% solution). The solution was heated for 10 minutes in an incubator at 60°C, and, then ultrasonicated for 20 minutes with a sonicator. The obtained solution was used for the experiment. Hereinafter, GSL-7 dissolved by the above method is abbreviated as GSL-7(P20).
Next, with respect to 0.1 mol of GSL-7, lipid compositions listed in the following table were each dissolved in a solvent (chloroform : methanol (volume ratio of 2 : 1)). Solutions were added to eggplant-shaped flasks and then the solvents were removed with a rotary evaporator at room temperature, so that lipid membranes were formed.
Physiological saline was added, the solutions were mixed using a vortex and then ultrasonication was carried out for 1 minute. Thus, liposomes were prepared.

(Lipid compositions)

Lipids used for liposome preparation were cholesterol (Chol, Wako), 1,2-dioleoyl-3-(trimethyl ammonium)propane (DOTAP, Avanti polar lipids), 3β-[N-(dimethylaminoethane)carbamoyl]cholesterol (DC cholesterol, Sigma), and 1,2-dioleoyl-sn-glycero-3-phosphatidylethanolamine (DOPE, Avanti polar lipids). Moreover, the amount of each lipid in the following table is represented by mol equivalent to 0.1 mol of GSL-7.

[Table 2]

| | Chol | DOTAP | DC cholesterol | DOPE |
|---|---|---|---|---|
| Liposome A | 2 | 2 | | |
| Liposome B | | 2 | | 2 |
| Liposome C | 1 | 2 | | 1 |
| Liposome D | 1.5 | 2 | | 0.5 |
| Liposome E | | | 2 | 2 |

(Measurement of capability for accelerating IFN-γ production)

Liposomes A to E prepared by the above method and GSL-7 (P20) not undergoing liposome formation were separately added to the splenocytes ($2 \times 10^6$) of C57BL/10ScSn mice (8- to 10-week-old males) (autogenic) and then IFN-γ levels in the culture supernatants were measured after 48 hours. The concentrations were employed so that the concentration of GSL-7 was 100 ng/ml. IFN-γ levels were measured by sandwich ELISA using purified anti-IFN-γ antibodies (R4-6A2) (Becton, Dickinson and Company, Japan) as capture antibodies and biotin-conjugated anti-IFN-γ antibodies (AN-18) (Becton, Dickinson and Company, Japan) as detection antibodies. After the reaction using the biotin-conjugated antibodies, alkaline phosphatase-labeled streptavidin (SIGMA) was conjugated thereto and then the color was developed using paranitrophenyl phosphate as a substrate. Absorbance at a wavelength of 405 nm was measured using MICROPLATE READER Model 550 (Biorad Laboratories, Japan). IFN-γ concentrations in test samples were calculated based on the standard curve of IFN-γ in the recombinant mice.
Comparative experiments were conducted as follows: (1) none of liposomes A to E and GSL-7 (P20) not undergoing liposome formation were added and the other procedures were carried out similarly (control); and (2) only GSL-7 (P20) not undergoing liposome formation was added and the other procedures were carried out similarly (GSL-7(P20)). The results are also shown below.

(Results)

The results are shown in the following tables. This experiment was separately carried out three times under the same conditions. The results obtained in the same experiment are collectively shown in the same table.
As shown in the tables below, even when splenocytes were stimulated with GSL-7 (P20) having a concentration of 100 ng/ml, IFN-γ production was barely induced to a level similar to that in the case of the control.
On the other hand, Liposome A and Liposome B significantly induced IFN-γ production from splenocytes. Particularly, Liposome B induced the same to an extremely significant degree.
In addition, regarding IFN-γ production levels, in the following tables, no significant difference was observed between the case in which GSL-7(P20) was added and the case in which the control was added. However, when the amount of GSL-7(P20) added was 1 μg/ml or more, significant differences were observed. Specifically, it was confirmed that the liposomes of the present invention can accelerate IFN-γ production in amounts significantly lower than that in the case of using GSL-7 not undergoing liposome formation.

[Table 3]

| | IFN-γ (pg/ml) |
|---|---|
| Control | 300.5 ± 64.3 |
| GSL-7(P20) | 243.3 ± 66.1 |
| Liposome A | 512.8 ± 184.0 |
| Liposome B | 2599.8 ± 744.0 |

[Table 4]

| | IFN-γ (pg/ml) |
|---|---|
| Control | 368.8 ± 24.2 |
| Liposome B | 2609.3 ± 95.3 |
| Liposome C | 1347.5 ± 182.6 |
| Liposome D | 747.5 ± 96.0 |

[Table 5]

| | IFN-γ (pg/ml) |
|---|---|
| Control | 484.3 ± 58.6 |
| Liposome B | 1648.4 ± 135.3 |
| Liposome E | 1357.5 ± 203.4 |

Example 2 Induction of IFN-γ production via alteration of liposome concentration

The concentration of Liposome B or GSL-7 (P20) of Example 1 was varied so that the concentration of GSL-7 was as listed in the following table. The other concentrations were similarly varied and then IFN-γ concentrations in samples were separately calculated. Furthermore, only the lipid composition of Liposome B was added (not containing GSL-7) and the other procedures were similarly carried out.

(Results)

These results are shown in the following table.

[Table 6]

| Added material | Concentration (in terms of the concentration of GSL-7) | IFN-γ (pg/ml) |
|---|---|---|
| Only lipid composition of Liposome B | - | 174.0 ± 27.8 |
| Liposome B | 50 ng/ml | 370.7 ± 27.3 |
| Liposome B | 100 ng/ml | 1717.5 ± 352.1 |
| Liposome B | 500 ng/ml | 2239.5 ± 198.3 |
| GSL-7(P20) | 50 ng/ml | 204.3 ± 80.0 |
| GSL-7(P20) | 100 ng/ml | 162.7 ± 9.3 |
| GSL-7(P20) | 500 ng/ml | 279.9 ± 15.1 |
| GSL-7(P20) | 1000 ng/ml | 621.9 ± 194.3 |
| GSL-7(P20) | 5000 ng/ml | 1554.9 ± 508.3 |
| GSL-7(P20) | 10000 ng/ml | 2134.7 ± 347.1 |

As is clear from the above table, it was confirmed that IFN-γ induction is accelerated by increasing the amount of Liposome B added.
In addition, regarding the production amount of IFN-γ, when GSL-7(P20) with a concentration of 1 μg/ml or more was added, a significant difference was confirmed when compared with that of the control. It was confirmed that IFN-γ induction was accelerated by Liposome B to a level approximately 50 or more times higher in terms of specific activity.

Example 3 Influence of anti-CD1d mAb and isotype mAb on the effects of accelerating IFN-γ production

Liposome B in Example 2 was added so that the concentration of GSL-7 was 100 ng/ml, anti-CD1d mAb (BD Pharmingen) or isotype mAb (BD Pharmingen) was added so that concentration was 40 μg/ml in addition to the addition of Liposome B and the other procedures were carried out in the same manner as Example 2. Addition of isotype mAb was carried out to confirm that Liposome B activity was not inhibited by completely-unrelated other antibodies.

(Results)

These results are shown in the following table together with the result of a comparative example.

[Table 7]

| | IFN-γ (pg/ml) |
|---|---|
| Control | 442.4 ± 98.8 |
| Lipid composition of Liposome B | 307.3 ± 56.6 |
| Liposome B | 1468.0 ± 145.5 |
| Liposome B + anti-CD1d mAb | 694.3 ± 126.3 |
| Liposome B + isotype mAb | 1404.5 ± 67.8 |

It was confirmed by these results that Liposome B activity was significantly inhibited by the addition of anti-CD1d mAb. Furthermore, when isotype mAb was added, IFN-γ production due to the addition of Liposome B was never inhibited.

Example 4 IFN-γ induction in various mice

IFN-γ induction from splenocytes was measured in the same manner in Example 1, which, however, C57BL/10ScSn (WT) mice or C57BL/10ScN ($TLR4^{-/-}$ mice) were used instead of C57BL/10ScSn mice and Liposome B (in terms of the concentration of GSL-7: 500 ng/ml) was used.
Furthermore, 20 μg/ml of anti-IL-12 p40/p70 mAb (BD Pharmingen) was added simultaneously with addition of Liposome B and the other procedures were carried out in the same manner as Example 1
Furthermore, C57BL/10ScSn (WT) mice were used and 20 μg/ml of isotope mAb (R&D systems) was added simultaneously with addition of Liposome B. The other procedures were carried out in the same manner as Example 1.

(Results)

As shown in the following tables, IFN-γ production was accelerated by the addition of Liposome B when any mice were used, and the production amount of IFN-γ was significantly decreased by the addition of anti-IL-12 p40/p70 mAb. Furthermore, the addition of isotope mAb had no effect on the production amount of IFN-γ.

[Table 8]

| | | IFN-γ (pg/ml) |
|---|---|---|
| WT | Control | 347.6 ± 27.0 |
| | Liposome B | 1820.9 ± 151.7 |
| | Liposome B+anti-IL-12 p40/p70 mAb | 595.9 ± 66.2 |
| $TLR4^{-/-}$ | Control | 320.0 ± 54.1 |
| | Liposome B | 2052.6 ± 38.2 |
| | Liposome B+anti-IL-12 p40/p70 mAb | 562.8 ± 106.4 |

[Table 9]

| | IFN-γ (pg/ml) |
|---|---|
| Control | 383.4 ± 87.2 |
| Liposome B | 2428.2 ± 556 |
| Liposome B + isotype mAb | 2428.2 ± 265 |

Example 5 Induction of IL-12p40 production from splenocytes

(Measurement of capability of accelerating IL-12p40 production)

Liposome B prepared by the method described in Example 1 and GSL-7(P20) not undergoing liposome formation were separately added to splenocytes ($2 \times 10^6$) of C57BL/10ScSn (WT) mice (autogenic). After 24 hours, IL-12p40 levels in the culture supernatants were measured. Concentrations employed herein were adjusted so that the concentrations of GSL-7 were as those listed in the following table. IL-12p40 levels were measured using an OptEIA mouse cytokine detection set (BD Bioscience). The other procedures were carried out according to the measurement of IFN-γ in Example 1.

(Results)

The results are shown in the following table.

[Table 10]

| Added material | Concentration in terms of the concentration of GSL-7 | IL-12 p40 (pg/ml) |
|---|---|---|
| Only lipid composition of Liposome B | - | 67.9 ± 55.5 |
| Liposome B | 50 ng/ml | 129.1 ± 83.8 |
| Liposome B | 100 ng/ml | 258.2 ± 33.3 |
| Liposome B | 500 ng/ml | 498.2 ± 72.9 |
| GSL-7(P20) | 50 ng/ml | 81.5 ± 72.1 |

(continued)

| Added material | Concentration in terms of the concentration of GSL-7 | IL-12 p40 (pg/ml) |
|---|---|---|
| GSL-7(P20) | 100 ng/ml | 95.1 ± 53.5 |
| GSL-7(P20) | 500 ng/ml | 74.7 ± 49.9 |
| GSL-7(P20) | 1000 ng/ml | 47.5 ± 36.3 |
| GSL-7(P20) | 5000 ng/ml | 39.6 ± 33.8 |
| GSL-7(P20) | 10000 ng/ml | 98.9 ± 39.6 |

It was confirmed that IL-12p40 production was significantly accelerated by the addition of Liposome B. Furthermore, it was confirmed that the production amount of IL-12p40 was increased by increasing the amount of Liposome B added.

Example 6 Influence of anti-CD1d mAb or isotype mAb on the effects of accelerating IL-12 production

As in the case of Example 5 in which Liposome B was added so that the concentration of GSL-7 was 500 ng/ml, anti-CD1d mAb (BD Pharmingen) or isotype mAb was added simultaneously with the addition of Liposome B so that the concentration of the mAb was 40 μg/ml. The other procedures were similarly carried out.

(Results)

The results are shown in the following table together with the result of a comparative example.

[Table 11]

| | IL-12 p40 (pg/ml) |
|---|---|
| Control | 466.4 ± 88.2 |
| Lipid composition of Liposome B | 661.8 ± 108.1 |
| Liposome B + anti-CD1d mAb | 579.9 ± 176.5 |
| Liposome B + isotype mAb | 2117.9 ± 121.6 |

It was confirmed that IL-12 p40 induction from splenocytes due to the addition of Liposome B was significantly decreased by the addition of anti-CD1d mAb (BD Pharmingen). On the other hand, when isotype mAb was added, IL-12 p40 induction by Liposome B was not inhibited.

Example 7 Effects of accelerating IL-12 production in various mice

C57BL/10ScSn (WT) mice and C57BL/10ScN ($TLR4^{-/-}$ mice) were used instead of C57BL/10ScSn mice in Example 5. Liposome B was added so that the concentration of GSL-7 was 500 ng/ml. Then IL-12 induction from splenocytes was measured in the same manner as Example 5.

(Results)

The results are shown in the following table together with the result of a comparative example.

[Table 12]

| | | IL-12 p40 (pg/ml) |
|---|---|---|
| WT | Control | 348.5 ± 198.8 |
| | GSL-7(P20) | 377.1 ± 163.2 |
| | Liposome B | 1284.2 ± 167.3 |

(continued)

| | | IL-12 p40 (pg/ml) |
|---|---|---|
| TLR4$^{-/-}$ | Control | 205.3 ± 93.5 |
| | GSL-7(P20) | 243.5 ± 152.0 |
| | Liposome B | 1102.8 ± 227.3 |

As is clear from the following table, it was confirmed that the effects of inducing IL-12 was significantly improved by the use of Liposome B.

Example 8 Amounts of liposomes introduced by CD11c$^+$ dendritic cells prepared from splenocytes

The amounts of liposomes introduced by CD11c$^+$ dendritic cells prepared from splenocytes were confirmed. Dendritic cells were prepared by separating the cells from the splenocytes of C57BL/10ScSn mice (8- to 10-week-old males) using magnetic bead-bound anti-CD11c mAb (Miltenyi Biotec). Fluorescently labeled Liposome B and Liposome D were added. At 90 minutes after addition, the amount of each liposome induced by dendritic cells was examined using a flow cytometer (Beckman Coulter).

As shown in Fig. 1, no difference was observed between the amount of Liposome B (continuous line) induced and the amount of Liposome D (broken line) induced. In addition, in Fig. 1, the vertical axis denotes the numbers of cells and the transverse axis denotes fluorescence intensity.

Example 9 Effects of dendritic cell activation (1)

One (1) μg of GSL-7(P20) or Liposome B (GSL-7 content: 1 μg) was administered to C57BL/6 mice (8- to 10-week-old females) through the caudal vein. After 16 hours, splenectomy was carried out. A cell suspension was prepared from the spleen extracted. Splenocytes were treated with anti-FcγR antibodies (2.4G2) and then PE-labeled anti-CD11c antibodies, biotin-labeled anti-CD80 antibodies, and biotin-labeled anti-CD86 antibodies were added. After addition of the antibodies to cells, the reaction was allowed to proceed at 4°C in the dark for 30 minutes. The cells treated with the biotin-labeled antibodies were allowed to react with Cy-chrome-bound streptavidin at 4°C in the dark for 30 minutes. Measurement was carried out using EPICS ELITE ESP.

These results are shown in the following table together with the result of a comparative example.
As shown in the following table, the degree of NKT cell activation was found to be higher in the case of administration of GSL-7 (P20) than that in the case of administration of the control. Furthermore, it was confirmed that administration of Liposome B containing 1 μg of GSL-7 resulted in even higher degree of dendritic cell activation.

[Table 13]

| | CD80 | CD86 |
|---|---|---|
| Control | 71.5 + 2.1 | 59.2 ± 1.7 |
| GSL-7(P20) | 76.6 ± 0.9 | 62.1 ± 0.9 |
| Liposome B | 79.4 ± 1.3 | 64.2 ± 0.6 |

Example 10 Effects of dendritic cell activation (2)

Liposome B (GSL-7 content: 1 μg) was administered to C57BL/6 mice (8- to 10-week-old females) through the caudal vein. After 2 hours, splenectomy was carried out. Splenocytes were treated with anti-FcγR antibodies (2.4G2) and then FITC-labeled anti-CD11c antibodies, PE-labeled anti-CD8α antibodies, and biotin-labeled anti-CD40 antibodies were added. After addition of the antibodies to cells, the reaction was allowed to proceed at 4°C in the dark for 30 minutes. The cells treated with the biotin-labeled antibodies were allowed to react with Cy-chrome-bound streptavidin at 4°C in the dark for 30 minutes. Measurement was carried out using EPICS ELITE ESP.

The results are shown in the following table together with the result of a comparative example.
As shown in the following table, a significant increase in CD40 expression was observed in CD8α$^+$ cells by the addition of GSL-7 and an even higher increase in expression was observed by the addition of Liposome B.

[Table 14]

| | CD40(%) |
|---|---|
| Control | 56.1 ± 2.0 |
| GSL-7(P20) | 63.6 ± 3.0 |
| Liposome B | 74.3 ± 0.3 |

Example 11 Induction of *in* vivo cytokine production

(Measurement of capability for accelerating IFN-γ production and capability for accelerating IL-4 production)

GSL-7(P20) (10 μg) or liposome B (in an amount so that the GSL-7 content was 10 μg) prepared as in Example 1 was administered to C57BL/6 mice (8- to 10-week-old females) through the caudal vein. After 16 hours, serum IFN-γ and IL-4 concentrations were measured using an *in vivo* capture assay kit (Becton, Dickinson and Company, Japan).

(Results)

The results are shown in the following table together with the result of a comparative example.

[Table 15]

| | IFN-γ (pg/ml) | IL-4 (pg/ml) |
|---|---|---|
| Control | 246.2 ± 29.4 | 101.6 ± 14.5 |
| GSL-7 (P20) | 2704.5 ± 321.8 | 5290.9 ± 1000.8 |
| Liposome B | 6337.9 ± 536.9 | 10377.0 ± 1190.8 |

Administration of GSL-7(P20) or Liposome B significantly induced IFN-γ production and IL-4 production. In particular, it was confirmed that administration of Liposome B induced such production to a level more extremely significantly than that induced by administration of GSL-7(P20) not undergoing liposome formation.

Example 12 Effects of NTK cell activation (1)

(Measurement of NKT cell activation)

GSL-7(P20) (1 μg) or Liposome B (in an amount so that the GSL-7 content was 1 μg) prepared as in Example 1 was administered to C57BL/6 mice (8- to 10-week-old females) through the caudal vein. After 16 hours, the liver and the spleen were excised. Cell suspensions were prepared from the thus excised liver and spleen. A cell suspension from the liver was subjected to specific gravity centrifugation using 45% Percoll and 67.5% Percoll, so that intrahepatic mononuclear cells were obtained. Intrahepatic mononuclear cells and splenocytes were treated with anti-FcγR antibodies (2.4G2) (Becton, Dickinson and Company, Japan) and then PE-labeled anti-NK1.1 antibodies (Becton, Dickinson and Company, Japan) and biotin-labeled anti-TCRαβ antibodies (Becton, Dickinson and Company, Japan) were added. After addition of the antibodies to cells, the reaction was allowed to proceed at 4°C in the dark for 30 minutes. The cells treated with the biotin-labeled antibodies were allowed to react with Cy-chrome-bound streptavidin (Zymed) at 4°C in the dark for 30 minutes. Measurement was carried out using EPICS ELITE ESP.
In addition, it is known that when NTK cells are activated, NK1.1 molecules on the cell surface are down-regulated. Hence, the NK1.1 molecule expression was examined as an indicator of NKT cell activation by administration of GSL-7(P20) or Liposome B.

(Results)

The results are shown in the following table together with the result of a comparative example.

[Table 16]

| | Liver | Spleen |
|---|---|---|
| Control | 30.4 ± 0.6 | 2.1 ± 0.4 |
| GSL-7(P20) | 33.9 ± 2.2 | 1.6 ± 0.1 |
| Liposome B | 19.1 ± 2.7 | 1.3 ± 0.3 |

It was confirmed that addition of Liposome B resulted in significantly enhanced NKT cell activation compared with the case of administration of an equivalent amount of GSL-7(P20).
Furthermore, as shown in the following table, concerning NKT cell activation, no significant difference was observed between the case of adding the control and the case of adding GSL-7(P20); however, a significant difference was observed between the same when the amount of GSL-7(P20) to be added had been increased.

Example 13 Effects of NTK cell activation (2)

Liposome B (GSL-7 content: 1 μg) was administered to C57BL/6 mice (8- to 10-week-old females) through the caudal vein. After 2 hours, splenectomy was carried out. Splenocytes were treated with anti-FcγR antibodies (2.4G2) and then FITC-labeled anti-TCRβ antibodies and biotin-labeled anti-CD40L antibodies were added. After addition of the antibodies to cells, the reaction was allowed to proceed at 4°C in the dark for 30 minutes. The cells treated with the biotin-labeled antibodies were allowed to react with Cy-chrome-bound streptavidin at 4°C in the dark for 30 minutes. Measurement was carried out using EPICS ELITE ESP.

(Results)

The results are shown in the following table together with the results from a comparative example.
As shown in the table, it was confirmed that an increased CD40L expression level was increased in NKT cells by the administration of Liposome B.
[Table 17]

| | CD40L |
|---|---|
| Control | 9.7 ± 0.4 |
| Liposome B | 17.8 ± 0.5 |

Example 14 Effects of enhancing antibody production

(Preparation of ovalbumin-encapsulated liposome)

An ovalbumin (OVA) (SIGMA)-encapsulated liposome (Liposome F) was prepared.
The lipid composition is as follows: to 0.1 mols of GSL-7, 0.1 mols of (normal)_1,2-dipalmitoyl-sn-glycero-3-phosphoethanolamine-N-[methoxy (polyethyleneglycol)-2000] (Avanti polar lipids), 2 mols of DOTAP, and 2 mols of DOPE.
An ovalbumin (OVA) solution (100 μg/ml) was added to lipid membranes prepared by the method for liposome preparation described in Example 1. The mixture was mixed with a vortex, subjected to 1 minute of ultrasonication, and thus Liposome F was obtained.

(Measurement of the effects of enhancing antibody production)

Liposome F (in an amount so that 25 μg of GSL-7 and 25 μg of OVA were contained) was administered to C57BL/6 mice (8- to 10-week-old females) through the caudal vein. After 14 days, blood was collected and then serum anti-OVA antibody levels were measured by ELISA. An anti-OVA antibody titer (IgG2a) was assayed by adding the gradually diluted serum to a 96-well plate coated with OVA. Alkaline phosphatase (AP)-labeled anti-mouse IgG2a antibodies were used as detection antibodies. The color was developed using paranitrophenylphosphate (p-nitrophenyl phosphate) as a substrate. The reaction was terminated with 3N NaOH, the absorbance at 405 nm was measured using a microplate reader.

(Results)

The results are shown in the following table together with the result of a comparative example. As shown in the following table, the degree of enhancement in anti-OVA antibody production was observed to be higher in the case of administration of Liposome F than that in the case of administration of the mixed solution of GSL-7(P20) not undergoing liposome formation and OVA.

[Table 18]

| | Anti-OVA IgG2a |
|---|---|
| Control | 0.034 ± 0.010 |
| OVA | 0.049 ± 0.018 |
| GSL-7(P20) + OVA mixed solution | 0.053 ± 0.012 |
| Liposome F | 0.212 ± 0.028 |

Example 15 Induction of antigen-specific IFN-γ production from splenocytes

Liposome F (prepared in an amount so that 25 μg of GSL-7 was contained) prepared as in Example 14 was administered to C57BL/6 mice (8-to 10-week-old males) through the caudal vein. After 1 week, the spleen was excised. Splenocytes ($5 \times 10^5$) were stimulated with OVA (1 mg/ml). After 96 hours, IFN-γ levels in the culture supernatants were measured. IFN-γ levels were measured by sandwich ELISA using purified anti-IFN-γ antibodies (R4-6A2) as capture antibodies and biotin-labeled anti-IFN-y antibodies (AN-18) as detection antibodies. In the subsequent reaction, alkaline phosphatase-labeled streptavidin was caused to bind to biotin-labeled antibodies. The color was developed using p-nitrophenyl phosphate as a substrate. The absorbance at a wavelength of 405 nm was measured using a microplate reader (MICROPLATE READER Model 550). IFN-γ concentrations in test samples were calculated based on the standard curve of IFN-γ in the recombinant mice.

(Results)

The results are shown in the following table together with the result of a comparative example. As shown in the following table, IFN-γ production from splenocytes was enhanced in the case of administration of Liposome F compared with the case of administration of the mixed solution of GSL-7(P20) not undergoing liposome formation and OVA.

[Table 19]

| | IFN-γ (ng/ml) |
|---|---|
| Control | 0.58 ± 0.05 |
| OVA | 4.79 ± 0.45 |
| GSL-7(P20)+OVA | 7.20 ± 1.63 |
| Liposome F | 15.9 ± 3.73 |

Example 16 Effects of enhancing antitumor immunity

Tumor cells ($3 \times 10^6$ per mouse) expressing OVA; that is, E.G7-OVA cells, were subcutaneously inoculated as antigens to the flanks of C57BL/6 mice (8- to 10-week-old males). After 1 week, when each tumor diameter reached 8 mm to 10 mm, a mixed solution of GSL-7(P20) (25 μg) and OVA (25 μg) or Liposome F (GSL-7 content: 25 μg and OVA (25 μg)) prepared as in Example 14 was administered through the caudal vein. After 20 days, tumor sizes were measured. As a control, a control liposome prepared by removing GSL-7 from the composition of Liposome F was used. Tumor volumes were calculated by the following Formula.

$$\text{Tumor volume} = 0.5 \times \text{length} \times (\text{width})^2$$

The results are shown in the following table. As is clear from the following table, tumor shrink was observed because of the addition of GSL-7(P20)+OVA and significant tumor shrink was observed because of the addition of Liposome F.

[Table 20]

| | Tumor volume ($mm^3$) |
|---|---|
| Control | 2802.2 ± 620.0 |
| Control liposome | 2182.7 ± 415.8 |
| GSL-7(P20)+OVA | 982.1 ± 440.1 |
| Liposome F | 267.2 ± 209.5 |

Furthermore, these photographs are shown in Fig. 2.

In Fig. 2, from the first row, the results of the control, administration of the control liposome, administration of the mixed solution of GSL-7(P20) and OVA, and administration of Liposome F are shown in sequence. Tumor shrink was observed as a result of the addition of GSL-7(P20)+OVA and significant tumor shrink was observed as a result of the addition of Liposome F.

Moreover, survival (%) of the mice on day 60 after inoculation of tumor was measured. The results are shown in the following table.

[Table 21]

| | Survival (%) |
|---|---|
| Control | 0 |
| Control liposome | 0 |
| GSL-7(P20)+OVA | 40 |
| Liposome F | 80 |

As is clear from the above table, significantly high survival % of the mice was confirmed as a result of the addition of Liposome F.

Example 17 Induction of IFN-γ production 2

The experiment was carried out in the same manner as that in Example 1 except that a liposome prepared by substituting GSL-7 contained in Liposome B with GSL-1 was used. The results are shown in the following table.

(Results)

As shown in the following table, even the use of the liposome in which GSL-7 of Liposome B had been substituted with GSL-1 was found to significantly induce IFN-γ production from splenocytes.

[Table 22]

| | IFN-γ (pg/ml) |
|---|---|
| Control | 234.6 ± 69.5 |
| Only lipid composition of Liposome B | 434.3 ± 143.5 |
| Liposome B(GSL-1) | 1511.5 ± 254.6 |

Example 18 Induction of IFN-γ production 3

The experiment was carried out in a manner similar to that in Example 1, except that a liposome prepared by

substituting GSL-7 contained in Liposome B with GSL-2, GSL-6, or GSL-13 was used. All of these liposomes were found to have tendencies of inducing IFN-γ production.

Example 19 Observation of liposome

Liposome B was negatively stained with a 2% phosphotungstic acid (PTA) stain. Morphological observation was carried out using a transmission electron microscope (Hitachi H-8100, transmission electron microscope, acceleration voltage: 100 kV).
Furthermore, 1 mL of ethyl acetate was added to 0.5 mL of Liposome B (in terms of the concentration of GSL-7: 100 μg/mL). The solution was mixed vigorously and then subjected to 3 minutes of sonication with a sonicator. The solution was centrifuged (3000 RPM, 5 minutes, room temperature) and then the ethyl acetate layer that was the upper layer was collected in a *nasu* flask (eggplant-shaped flask). This procedure was repeated 3 times. The ethyl acetate collected with a rotary evaporator (35°C) was evaporated to dryness, so that a lipid extract was obtained. One (1) mL of ethyl acetate was added to the thus dried lipid extract and then the resultant was subjected to 5 minutes of sonication with a sonicator. The thus obtained lipid solution was developed with the use of TLC (Merck, Silica gel TLC aluminum plate, developing solvent C : M : acetic solution : water = 60 : 45 : 1 : 8 (volume ratio)) and then detection was confirmed by spraying with sulfuric acid followed by heating. Moreover, the experiment was similarly carried out using GSL-7 instead of Liposome B.

(Results)

The results of morophological observation under an electron microscope are shown in Figs. 3 and 4. Here, Fig. 4 shows an enlarged photograph of one of the liposomes in Fig. 3. It was confirmed based on these photographs that Liposome B contained lipids with a multi-lamellar structure and had a diameter approximately between 50 nm and 100 nm.
The results when lipids obtained by the use of Liposome B and lipids obtained by the use of GSL-7 were separately TLC-developed are shown in Fig. 5. Here, Fig. 5 A shows TLC-developed lipids obtained by the use of GSL-7 and Fig. 5 B shows TLC-developed lipids obtained by the use of Liposome B. One of the lipid spots obtained by the use of Liposome B was the same as that obtained by the use of GSL-7 in terms of mobility and tendency of color development after spraying with 10% sulfuric acid followed by heating. Thus, the spot was confirmed to be GSL-7.

Example 20 Tumor metastasis

Tumor cells (B16-F10, Institute for Genetic Medicine, Hokkaido University) were implanted in C57BL/6 mice (7-week-old females) through the caudal vein so that the number of cells was 2 x $10^5$ per mouse. Liposome B prepared in Example 1, or, GSL-7(P20) not undergoing liposome formation was dissolved in polysorbate and was administered to the mice intraperitoneally on days 1, 5, 9, and 13 after tumor cell implantation. The doses of GSL-7 were as shown in the following table. The mice were subjected to dissection on day 14 after implantation, and the number of lung sites of metastases was determined. Polysorbate alone or the lipid composition alone of Liposome B was administered followed by the same procedures. The results are shown in the following table.

(Results)

As shown in the following table, the number of lung sites of metastases was significantly decreased by administration of Liposome B.

[Table 23]

| | Dose of GSL-7 (μg) | Number of sites of metastases |
|---|---|---|
| Polysorbate | 0 | 192±72 |
| Lipid composition of Liposome B | 0 | 174±95 |
| GSL-7(P20) | 1 | 123±63 |
| GSL-7(P20) | 10 | 130±67 |
| Liposome B | 1 | 65±38 |
| Liposome B | 10 | 40±30 |

Industrial Applicability

According to the present invention, GSLs are caused to form liposomes, so as to make it possible to enhance various capabilities of GSLs related to immunopotentiating activity. Therefore, the GSLs of the present invention are more useful as agents with immunopotentiating activity than conventional GSLs.
In particular, the GSLs according to the present invention are extremely effective in that even a small amount of the GSL can result in exertion of an effect of immunopotentiating activity although the same amount of a conventional GSL exerts no effect of immunopotentiating activity.
Furthermore, the liposomes of the present invention are advantageous in that they undergo nano- and microcapsulation of GSLs. Specifically, through adjustment of the particle sizes, the lipid compositions, the electric charge, and the like of the liposomes, the *in vivo* behavior thereof can be controlled. Hence, the liposomes can be more efficiently delivered to target organs or tissue lesions than is possible with direct administration of GSLs. Therefore, the utility value of the liposomes of the present invention as medical substances are higher.
Further, the liposomes of the present invention are useful from the viewpoint of having few side effects because the toxicities thereof are low and endotoxin tolerance is not induced.

## Claims

1. A liposome, comprising at least one type of glycosphingolipid represented by the following Formula (1) inside and/or outside a lipid membrane:

Formula (1)

[Formula 1]

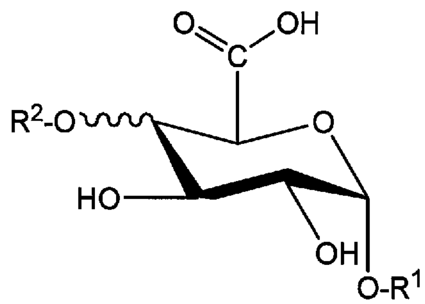

(wherein $R^1$ represents Formula (1-1):
Formula (1-1)

[Formula 2]

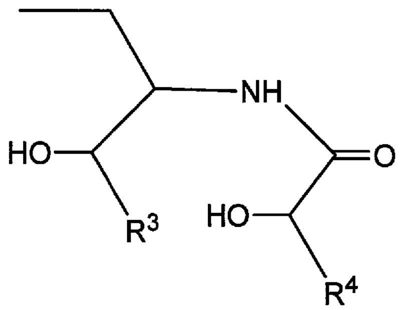

(wherein $R^3$ represents alkyl group or alkenyl group and $R^4$ represents alkyl group) and $R^2$ represents a hydrogen atom, α-galactose, α-glucose, α-mannose, α-glucosamine, β-glucosamine, or a combination of one

or more thereof.)

2. The liposome according to Claim 1, wherein $R^2$ in Formula (1) represents a hydrogen atom, α-galactose, α-glucose, α-mannose, α-glucosamine, β-glucosamine, or a combination of two or three thereof.

3. The liposome according to Claim 1, wherein $R^2$ in Formula (1) represents a hydrogen atom or the following $R^{62}$, $R^{63}$, $R^{64}$, or $R^{65}$.

[Formula 3]

$R^{62}$ :

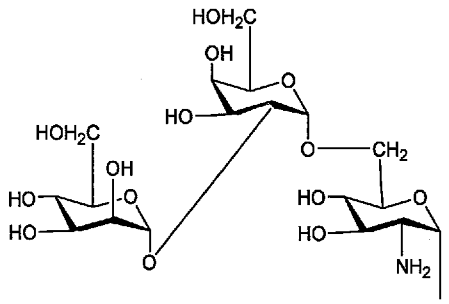

$R^{64}$ :

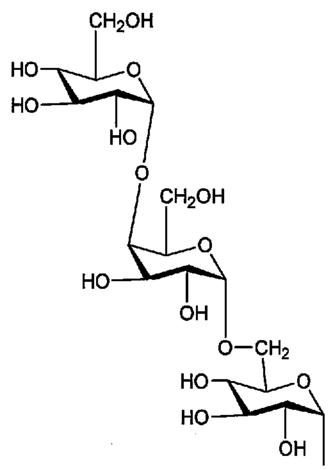

$R^{63}$ :

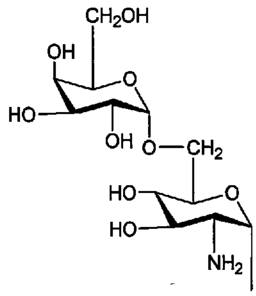

$R^{65}$ :

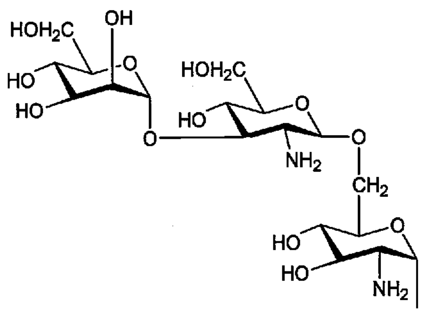

4. The liposome according to Claim 1, wherein $R^2$ in Formula (1) represents a hydrogen atom.

5. The liposome according to any one of Claims 1 to 4, wherein $R^1$ in Formula (1) represents the following $R^{51}$, $R^{52}$, $R^{53}$, $R^{54}$, $R^{55}$, $R^{56}$, $R^{57}$, $R^{58}$, $R^{59}$, $R^{70}$, $R^{71}$, $R^{72}$, $R^{73}$, $R^{74}$, $R^{75}$, $R^{76}$, $R^{77}$, or $R^{78}$.

[Formula 4]

$R^{51}$ :

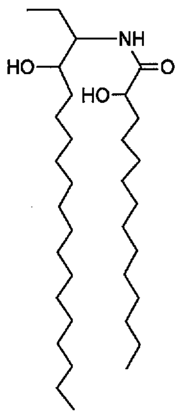

$R^{52}$ :

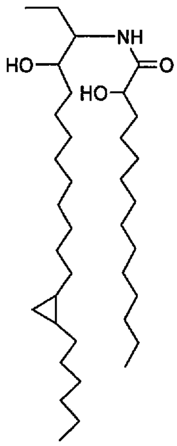

$R^{53}$ :

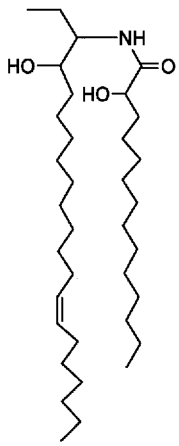

[Formula 5]

$R^{54}$ :

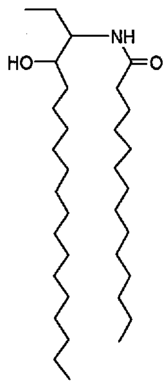

$R^{55}$ :

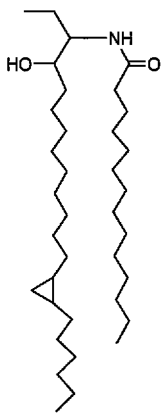

$R^{56}$ :

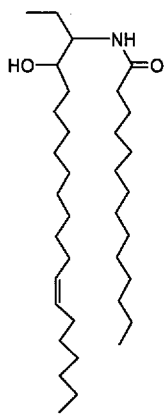

$R^{57}$ :

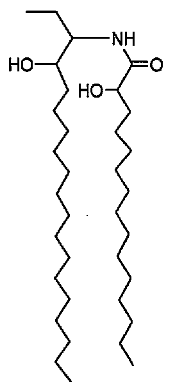

$R^{58}$ :

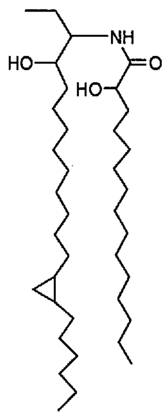

$R^{59}$ :

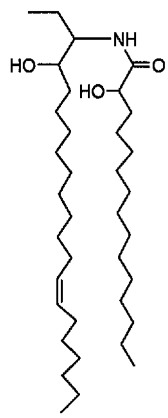

[Formula 6]

$R^{70}$ :

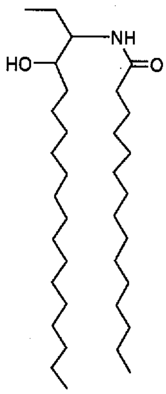

$R^{71}$ :

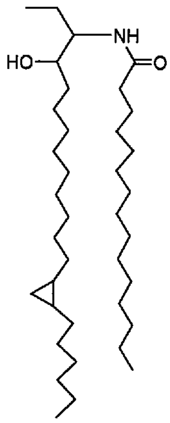

$R^{72}$ :

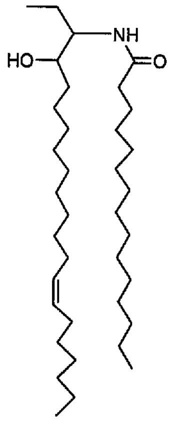

$R^{73}$ :

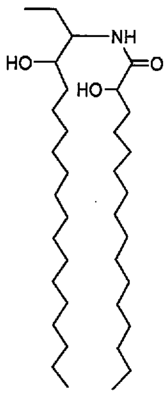

$R^{74}$:

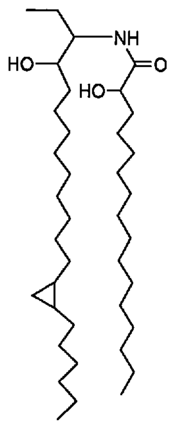

$R^{75}$ :

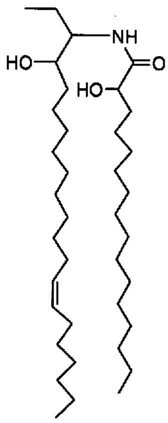

[Formula 7]

R76:

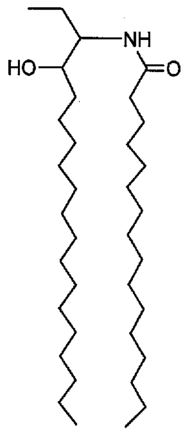

R77:

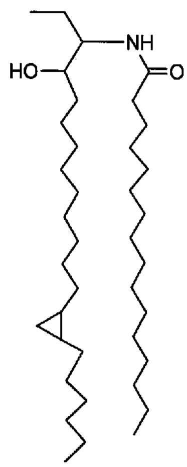

R78:

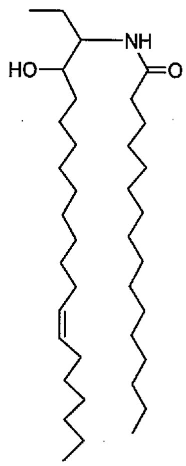

**6.** The liposome according to any one of Claims 1 to 5, wherein the lipid membranes is composed of a cationic lipid.

**7.** The liposome according to any one of Claims 1 to 5, wherein the lipid membrane is composed of a cationic lipid in the molar ratio of 0.4 or more.

**8.** The liposome according to Claim 6 or 7, wherein at least one type of the cationic lipid is 1,2-dioleoyl-3-(trimethyl ammonium)propane.

**9.** The liposome according to any one of Claims 1 to 7, wherein the lipid membrane is composed of 1,2-dioleoyl-3-(trimethyl ammonium)propane, 3β-[N-(dimethylaminoethane)carbamoyl]cholesterol, and 1,2-dioleoyl-sn-glycero-3-phosphatidylethanolamine in the molar ratio of 0.75 or more.

**10.** The liposome according to any one of Claims 1 to 9, further comprising an antigen inside and/or outside the lipid membrane.

**11.** An immunopotentiating composition, comprising the liposome according to any one of Claims 1 to 10.

**12.** A composition for accelerating IFN-γ production, comprising the liposome according to any one of Claims 1 to 10.

**13.** A composition for accelerating IL-4 production, comprising the liposome according to any one of Claims 1 to 10.

**14.** A composition for accelerating IL-12 production, comprising the liposome according to any one of Claims 1 to 10.

**15.** A composition for accelerating antibody production, comprising the liposome according to any one of Claims 1 to 10.

**16.** An antitumor composition, comprising the liposome according to any one of Claims 1 to 10.

**17.** A composition for NKT cell activation, comprising the liposome according to any one of Claims 1 to 10.

**18.** A composition for dendritic cell activation, comprising the liposome according to any one of Claims 1 to 10.

**19.** An antiviral composition, comprising the liposome according to any one of Claims 1 to 10.

**20.** An antiallergic composition, comprising the liposome according to any one of Claims 1 to 10.

Fig. 1

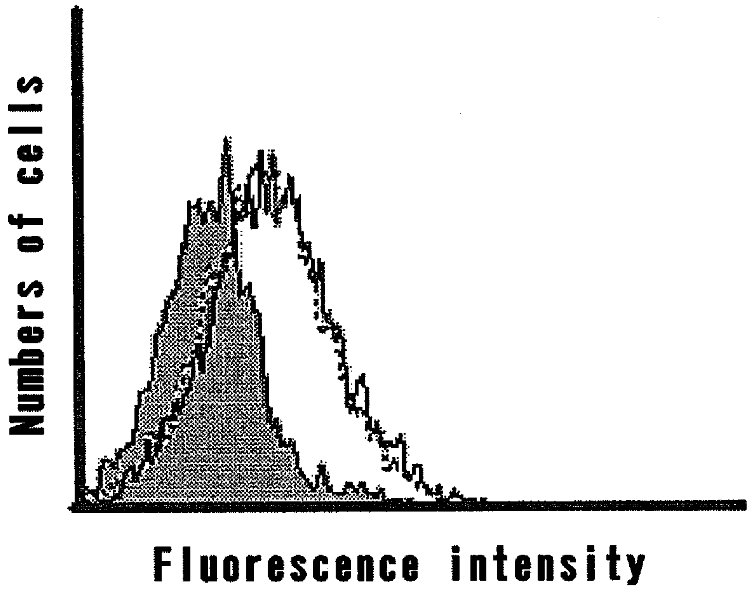
Numbers of cells
Fluorescence intensity

Fig. 2

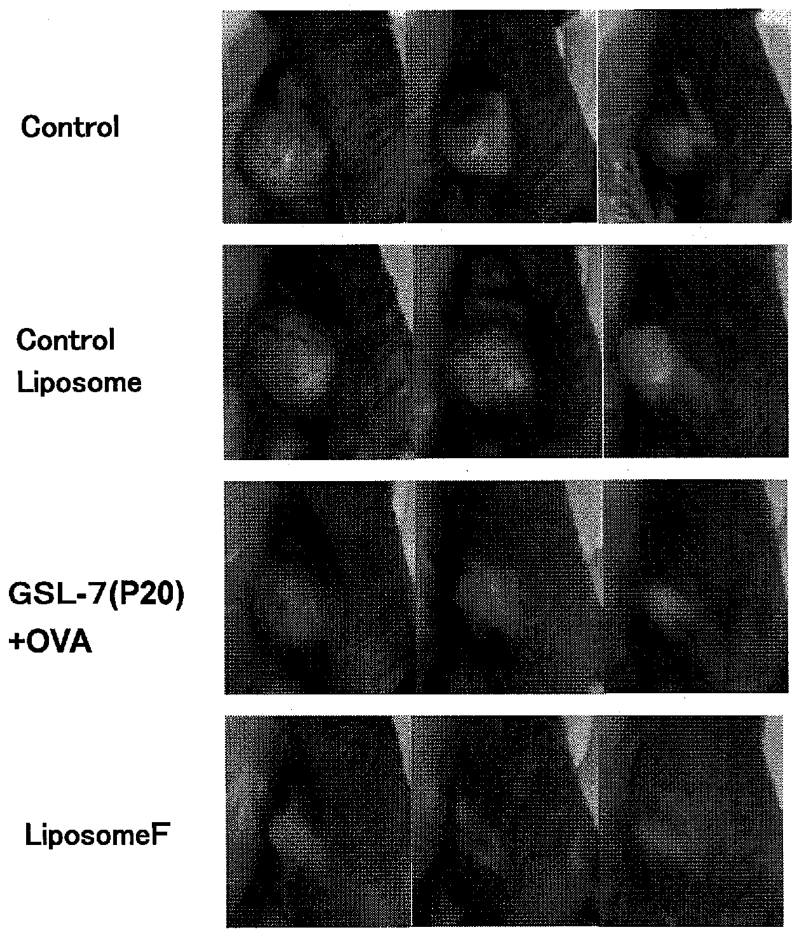
Control
Control Liposome
GSL-7(P20) +OVA
LiposomeF

Fig. 3

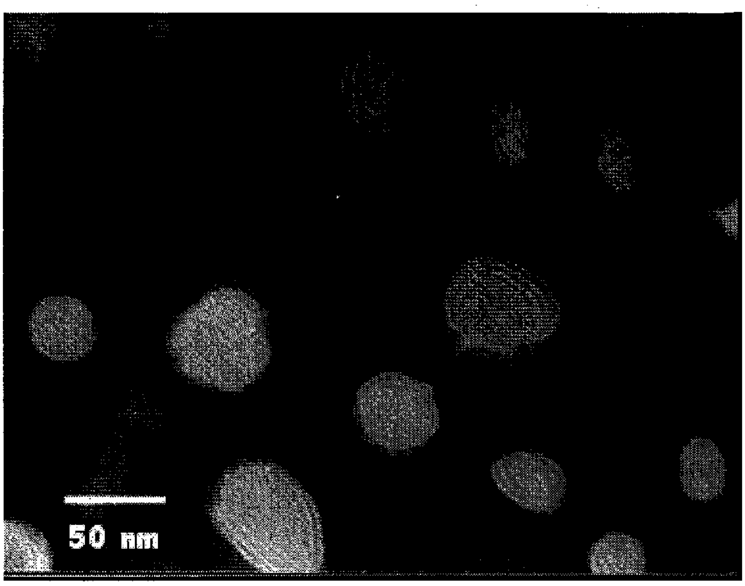


Fig. 4

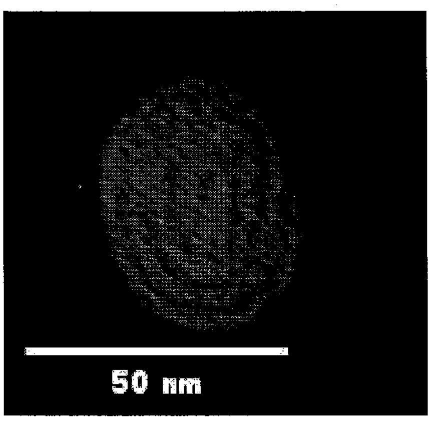

Fig. 5

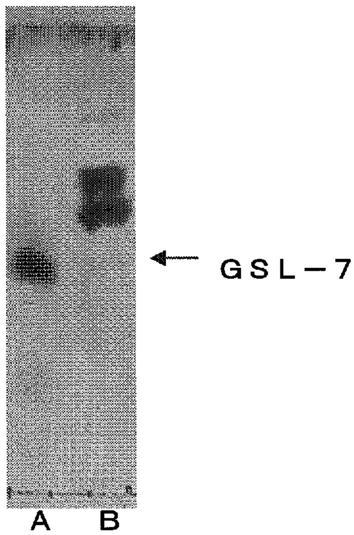


A：GSL－7

B：Liposome B

**INTERNATIONAL SEARCH REPORT**

International application No. PCT/JP2008/000692

A. CLASSIFICATION OF SUBJECT MATTER
*A61K47/36*(2006.01)i, *A61K9/127*(2006.01)i, *A61K39/00*(2006.01)i, *A61K39/35*(2006.01)i, *A61K47/18*(2006.01)i, *A61K47/24*(2006.01)i, *A61K47/28*(2006.01)i, *A61P35/00*(2006.01)i, *A61P37/04*(2006.01)i, *A61P43/00*(2006.01)i
According to International Patent Classification (IPC) or to both national classification and IPC

B. FIELDS SEARCHED

Minimum documentation searched (classification system followed by classification symbols)
A61K47/36, A61K9/127, A61K39/00, A61K39/35, A61K47/18, A61K47/24, A61K47/28, A61P35/00, A61P37/04, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
BIOSIS(STN), CAplus(STN), EMBASE(STN), MEDLINE(STN), JSTPlus(JDreamII), JMEDPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
|---|---|---|
| Y<br>A | JP 2005-263797 A (Kibun Food Chemifa Co., Ltd.),<br>29 September, 2005 (29.09.05),<br>Claims 1 to 26; Par. No. [0001]<br>& US 2006/0269524 A1 & EP 1716857 A1<br>& WO 2005/079813 A1 & CA 2526905 A | 1-5,10-20<br>6-9 |
| Y<br>A | WO 92/12986 A1 (Kabushiki Kaisha Kibun),<br>06 August, 1992 (06.08.92),<br>Claim 1; Technical Field<br>& JP 6-145189 A & US 5672693 A<br>& EP 639580 A1 & DE 69228128 C<br>& CA 2101076 A1 | 1-5,10-20<br>6-9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
25 April, 2008 (25.04.08)

Date of mailing of the international search report
13 May, 2008 (13.05.08)

Name and mailing address of the ISA/
Japanese Patent Office

Facsimile No.

Authorized officer

Telephone No.

Form PCT/ISA/210 (second sheet) (April 2007)

**INTERNATIONAL SEARCH REPORT**

International application No.
PCT/JP2008/000692

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | Maria H. A. Zanin et al, Physical characterization of surface-modified liposomes by incorporation of gangliosides designed for immunotherapies, Colloids and Surfaces A, 2004, Vol.251, No.1/3, p.175-182 | 1-5,10-20<br>6-9 |
| A | Andre Wiese et al, Planar asymmetric lipid bilayers of glycosphingolipid or lipopolysaccharide on one side and phospholipids on the other: membrane potential, porin function, and complement activation, Biophysical Journal, 1996, Vol.70, No.1, p.321-9 | 1-20 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 921298 A
- JP 11043437 A
- JP 2000051676 A
- JP 2002010797 A
- JP 2005263797 A
- WO 9212986 A


### Non-patent literature cited in the description


- *Microbiol. Immunol.,* 2000, vol. 44, 563-575
- **D. D. LASIC.** liposomes: from basic to applications. Elsevier Science Publishers, 1993, 1-171
- **BANGHAM, A.D. ; STANDISH, M.M. ; WATKINS, J.C.** Diffusion of univalent ions across the lamellae of swollen phospholipids. *J. Mol. Biol.,* 1965, vol. 13, 238-252